# EUROPEAN PATENT APPLICATION

(11) **EP 4 309 623 A1**
(43) Date of publication of application: **24.01.2024**
(21) Application number: 21931829.2
(22) Date of filing: 30.11.2021
(51) Int. Cl.: A61F 2/00, A61B 17/06, A61B 17/00

(54) **VARIABLE LIFTING THREAD FOR PAIN REDUCTION**

(30) Priority: 16.03.2021 KR 20210034281
(71) Applicant: Neo Dr. Inc., Wonju-si, Gangwon-do 26311 (KR)
(72) Inventor: KIM, Hyeon Ho, Seoul 05403 (KR)
(74) Representative: Caspary, Karsten
(86) International application number: PCT/KR2021/017838
(87) International publication number: WO 2022/196888

(57) **Abstract**

A suture thread for use in a lifting procedure according to the present invention is characterized by comprising: fixed portions composed of a non-stretchable medical thread and formed at both sides thereof to be fixed to the skin; and a variable portion which is positioned between the fixed portions and imparts structural elasticity, wherein the fixed portions and the variable portion are connected continuously, without a separate connecting structure, to form a single integrated medical thread.

## Description

### Technical Field

The present invention relates to a variable lifting thread for pain reduction, and more specifically, to a lifting thread having a variable portion with elasticity, which is firmly fixed without sagging of a lifted portion by using a typical medical thread lacking significant elasticity, and does not cause a pain in a treated area even when the muscles move significantly, such as during laughing or yawning, due to a variable portion formed at a portion of the medical thread to be structurally stretchable to prevent the medical thread from breaking.

### Background Art

Recently, with the growing interest in skin beauty, thread-embedding therapy, which is to remove skin sagging or wrinkles using medical threads, has been developed and is widely used.

The medical threads inserted through the thread-embedding therapy stimulate and tighten the surrounding skin tissues to enhance muscles and remove sagging skin or wrinkles, and are absorbed into the treated skin as time goes on.

Moreover, the threads used in the thread-embedding therapy are typically inserted using a thread inserter. The thread inserter includes a pipe-shaped insertion needle which is elongated in a back-and-forth direction and has a handle at the rear end, and a thread of which one end is inserted into the insertion needle and the other end is exposed to the outside and extends rearwards.

When the insertion needle is stuck into a human body, the thread is drawn into the body by the insertion needle. Once the thread reaches a desired position inside the body and the insertion needle is removed, only the thread remains inside the human body. The thread remaining inside the human body pulls and fixes the internal tissues to remove skin sagging and wrinkles.

On the other hand, as described above, in order to remove wrinkles or maintain skin tissue elasticity by inserting a thread into the human body, a larger diameter of the inserted thread yields more significant results. However, if the diameter of the insertion needle becomes larger, it causes a severe pain and potential side effects. Therefore, the diameter of the insertion needle is limited, and the thread which is inserted into the needle is also limited in diameter for use.

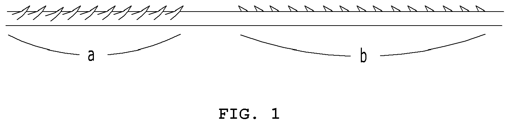

FIG. 1 illustrates a conventional straight-type suture 1 for a lifting procedure. The suture is divided into a first region and a second region centered around a specific point, and has a plurality of protrusions which are formed on circumferential portions of the first region (a) and the second region (b) to be inclined at an angle to face each other. The protrusions in the first region serve to lift the sagging skin, and the protrusions in the second region act as an anchor hold the lifted tissues not to sag again.

However, since such a conventional straight-type suture 1 for a lifting procedure only serves to pull the skin from beneath, the skin continuously maintains a rigid state, and a person who has the lifting procedure cannot naturally make facial expressions such as frowning or smiling. Furthermore, since most of the sutures are made of non-elastic materials, if the muscles that were treated move significantly, such as when yawning or laughing broadly, it may cause a pain in the treated area, tissues may tear, and in severe cases, the suture for lifting may break, so it reduces the lifting effect.

Therefore, the present invention aims to propose a new form of suture for a lifting procedure which can effectively address the problems of conventional sutures.

### [Cited References]

### [Patent Literature]

(Patent Document 1) Korean Patent No. 10-1969910 (April 11, 2019)

### Disclosure

### Technical Problem

Accordingly, the present invention has been made in view of the above-mentioned problems occurring in the related art, and it is an object of the present invention to provide a suture for a lifting procedure, which can firmly lift an area to be lifted without sagging by using a fixed portion with a non-stretchable structure, and can prevent a cause of a pain and prevent a thread from breaking even when muscles (or tissues) move significantly, as in laughing or yawning, by using a variable portion which is structurally stretchable in an area with a large movement.

In addition, the present invention aims to provide a suture for a lifting procedure, which can be easily manufactured using simple methods such as pressing, molding, laser processing, and ultrasonic machining, thereby reducing the production cost.

### Technical Solution

To accomplish the above-mentioned objects, according to the present invention, there is provided a variable lifting thread for pain reduction, which is formed to be partitioned in a length direction into a first region and a second region, wherein the first region includes a fixed portion with fixing grooves having an acute angled sloping jaw formed on the outer surface of the medical thread to penetrate into the subcutaneous tissue to be fixed, the second region includes a variable portion with elastic grooves formed alternately (zig-zag) on both sides of the outer circumference of the medical thread to have structural elasticity, and when an external force acts, a connection portion between the elastic grooves of the variable portion stretches to provide elasticity to the medical thread.

Between the variable portions of the second region, acute angled sloping jaws which can penetrate into the subcutaneous tissue to be fixed are additionally formed, and the fixing groove includes an inclined surface and the acute angled sloping jaw in an asymmetric structure.

Moreover, concavely curved grooves may be formed behind acute angled sloping jaw of the fixing groove to be spaced apart at a predetermined interval. When the acute angled sloping jaw penetrates into the subcutaneous tissue, the curved grooves are curved and rotated, so that the acute angled sloping jaw further protrudes in an outer circumferential direction of the medical thread to penetrate deeper into the subcutaneous tissue and become more firmly fixed.

The variable portion is formed in a spring shape by being formed spirally in the length direction of the medical thread. A depth (h) of the elastic groove is greater than a straight distance (H) from the center of the medical thread to the outer circumferential surface.

### Advantageous Effect

The suture for a lifting procedure according to the present invention, which is made with a single medical thread, includes the first region for firmly fixing a lifted portion to prevent sagging and the second region designed to be structurally stretchable and embedded in an area with a large movement, thereby being firmly fixed to the body tissue without sagging, and preventing pain and breaking of the thread even when muscles (or tissues) move significantly, such as during laughing or yawning.

Furthermore, the present invention can form a stretchable variable portion relatively simply using various methods, such as pressing, molding, laser processing, and ultrasonic machining, by using medical threads made of relatively inexpensive non-stretchable material, thereby reducing manufacturing costs.

Additionally, even if using a thread of the same diameter, since including the curved groove formed behind the acute angled sloping jaw to release bearing force to support the acute angled sloping jaw not to be pushed back, the acute angled sloping jaw is curved and rotated in the direction to fill the curved groove when penetrating into the body tissue and further protrudes outwardly to serve as an anchor, thereby fixing the lifted area more firmly.

### Description of Drawings

FIG. 1 illustrates a conventional suture used for a lifting procedure.
FIG. 2 schematically illustrates a variable lifting thread for pain reduction, which includes a first region and a second region, according to an embodiment of the present invention.
FIG. 3 is an enlarged view of the second region including a variable portion 300 and a fixed portion 200.
FIG. 4 is an enlarged view of the first region including the fixed portion 200.
FIG. 5 provides an enlarged view of a first region of a variable lifting thread for pain reduction according to another embodiment of the invention.
FIG. 6 is an enlarged view of the variable portion of the variable lifting thread for pain reduction according to a further embodiment of the invention.

### Mode for Invention

Hereinafter, Reference will be now made in detail to the preferred embodiments of the present invention with reference to the attached drawings. It will be understood that words or terms used in the specification and claims shall not be interpreted as the meaning defined in commonly used dictionaries. It will be further understood that the words or terms should be interpreted as having a meaning that is consistent with their meaning in the context of the relevant art and the technical idea of the invention.

In the entire specification of the present invention, when a part "includes" a component, unless there is a particular description contrary thereto, the part can further include other elements, not excluding the other elements.

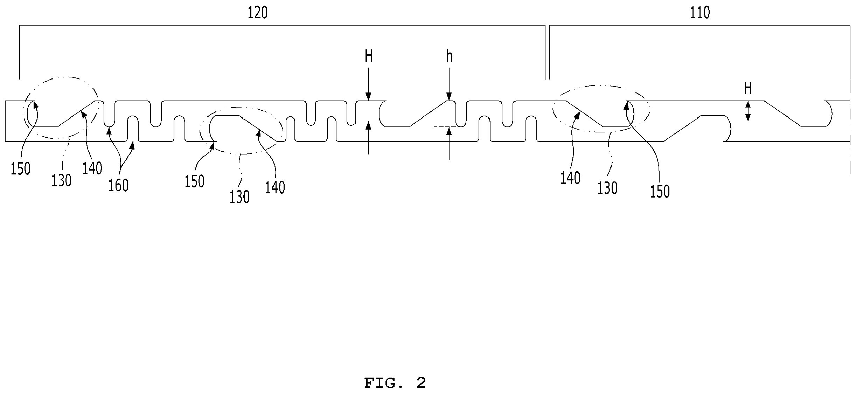

Hereinafter, referring to FIG. 2, a preferred embodiment of the present invention will be described. However, the scope of the present invention is not limited to the preferred embodiment described below, and those skilled in the art can make various modifications of the contents described in this specification within the scope of the claims of the present invention.

As illustrated in FIG. 2, a suture for a lifting procedure, namely a variable lifting thread for pain reduction, according to the present invention includes a first region 110 in which a plurality of fixed portions 200 are repeatedly formed, and a second region 120 in which fixed portions 200 and variable portions 300 are alternately and repeatedly formed.

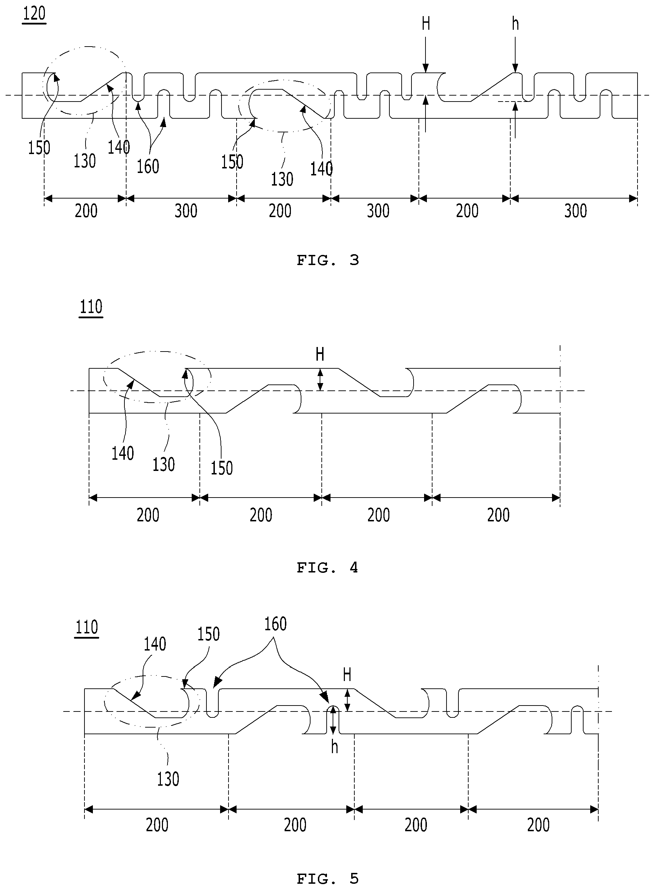

The fixed portions 200 formed in portions of the first region 110 and the second region 120 include a concave fixing groove 130 formed on the outer circumferential surface of a medical thread. It is preferable that the fixing groove 130 is asymmetrically formed, and includes a sloping surface 140 and an acute angled sloping jaw 150 (see FIG. 4).

The acute angled sloping jaw 150 formed on one side of the fixing groove 130 is designed as a sharp acute angled sloping jaw of less than 90 degrees, and serves to anchor into the body tissue and secure the tissue during the procedure.

Furthermore, the second region 120 has the variable portions which are formed on both sides of the medical thread and has zig-zagged U-shaped or V-shaped elastic grooves, such that a connection portion between the elastic grooves stretch like a spring when external force is applied, thereby having structural elasticity. In addition, between the variable portions, a fixing groove with an acute angled sloping jaw is also formed to dig into the body tissue of the lifted area and to firmly secure the tissue.

In addition, according to circumstances, the first region and the second region may be alternated along the length direction of the medical thread, and at least one or more of the first and second regions, and any combinations of the first and second regions are feasible and fall within the scope of the present invention.

Moreover, in a case in which the first regions having the fixing grooves are provided on both sides of the second region having the variable portions, the second region has only the variable portion, and the fixing groove may only be formed in the first region.

The acute angled sloping jaw 150 formed on one side of the fixing groove 200 can be manufactured on the outer circumferential surface of the medical thread through a method like ultrasonic or precision blade processing.

In addition, while the medical thread typically uses non-elastic materials, depending on circumstances, may be made of elastic materials as long as the first region has a fixed portion without a variable structure and the second region has a variable portion with a variable structure, thereby achieving the pain prevention and thread breakage prevention pursued by the present invention.

FIGS. 3 to 5 illustrate a straight-type thread with a circular cross-section. However, the thread may have a non-circular cross-sectional shape in order to increase the surface area and more effectively form protrusions. The non-circular cross-section may have a shape, such as oval, triangle, square, parallelepiped, trapezoid, rhombus, pentagon, hexagon, or cross shape. Since the medical thread is made by cutting a polymer filament formed by extrusion using a die with a circular cross-section, the cross-sectional shape of the extrusion die can be changed to a desired shape. Accordingly, the medial thread with a non-circular cross-section can be manufactured.

The medical thread (filament) used in the present invention can be made of biodegradable polymers, such as polydioxanone (PDO), polylactic acid (PLA), or polyglycolide (PGA).

As illustrated in FIG. 3, it is preferable for a depth (h) of the elastic groove 160 formed on the variable portion 300 to be greater than a straight distance (H) from the center of the filament, which is used to manufacture a suture for a lifting procedure, to the outer circumferential surface.

For the variable portion 300, as described above, a plurality of V-shaped or U-shaped curves or grooves are formed alternatively in the length direction and in a horizontal direction or in a vertical direction. The alternating structure provides structural elasticity to the non-stretchable medical thread.

As illustrated in FIG. 3, in the second region 120 of the suture for a lifting procedure according to the present invention, a variable portion 300 with V-shaped or U-shaped elastic grooves 160 alternating in the horizontal direction is formed between a pair of fixed portions 200, and the alternating pattern may be a basic alternating unit (U), and the variable portion 300 may have a plurality of basic alternating units (U) of the variable portion 300.

In another embodiment of the invention, as illustrated in FIG. 5, behind the fixed groove 130, curved grooves 160 are formed at predetermined intervals to release bearing force of the acute angled sloping jaw. Accordingly, when the acute angled sloping jaw is inserted into the body tissue, the acute angled sloping jaw is bent and rotates in a direction to fill the curved groove 160, and then, further protrudes outwardly, so penetrates deeper into the body tissue to firmly fix the tissue.

Such curved grooves 160 of the fixed portion 200 can be selectively applied to the suture for a lifting procedure according to the present invention.

In yet another embodiment of the invention, the variable portion 300 may be manufactured by helically carving in the length direction of the medical thread 100. In a case in which the variable portion 300 is formed in such a way that elastic grooves are formed helically in the length direction of the medical thread 100 and a connection portion between the elastic grooves is formed in a spring shape, a structural elasticity may be provided effectively even if non-stretchable thread is used.

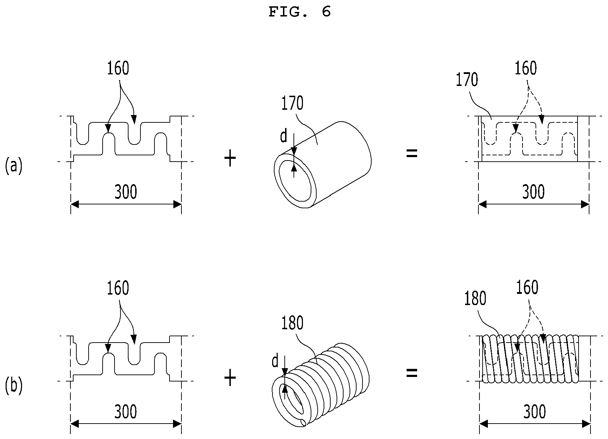

Meanwhile, in a still further embodiment of the present invention, as illustrated in Fig. 6, it is preferable to install a cover cylinder 170 on the outer circumference of the variable portion 300. In the case of the variable portion 300 without the cover cylinder, as described above, the plurality of V-shaped or U-shaped curves or grooves formed to alternate in the horizontal or vertical direction in the length direction ensure that the medical thread does not break due to the structural elasticity where the thread is stretched by the curves or grooves, even when muscles move significantly, such as when laughing or yawning. However, when laughing or yawning, if the curves or grooves are pulled and stretched, there is possibility to cause pain due to tissue irritation or damage caused by relative motion between the curves or grooves of the thread and the subcutaneous tissue. To minimize tissue irritation or damage, as illustrated in FIG. 6, a cover cylinder 170 is installed on the outer circumference of the variable portion 300. Then, even when the curves or grooves are pulled and stretched, the curves or grooves of the thread do not get in direction contact with the subcutaneous tissue but are stretched inside the cover cylinder, thereby preventing stimulation and damage of the tissue.

Here, the cover cylinder 170 is formed of a material used for manufacturing typical medical suture.

The cover cylinder 170 may be formed into a tension spring-type elastic thread cover tube 180 by being wound with an elastic thread. The elastic thread cover tube 180 can be manufactured by winding the medical thread tightly in a spiral shape around a cylinder of a predetermined diameter (e.g., an insertion needle) and heating the elastic thread with a heating device to form a tension spring shape (cylinder shape) with elasticity. Such a heating device may be an oven, a thermal dryer, or a hairdryer. The heating temperature is preferably in a range of 60% to 99% of the melting point of the medical thread. The medical thread thermally treated is enhanced in elasticity even though the cylinder is removed, so the elastic thread cover tube 180 tightly wound in the spiral shape can be maintained like a tension spring as it is without being released.

At this time, it is preferable that the radius of the cover cylinder 170 and the elastic thread cover tube 180 is the same as or smaller than a radius (H) of the lifting thread. When manufacturing the variable portion 300 of the medical thread for a lifting procedure, it is necessary to reduce the radius of the variable portion 300 by the thickness (d) of the cover cylinder 170 or the elastic thread cover tube 180. In other words, it is preferable to form the distance from the center of the medical thread to the outer circumference of the variable portion 300 as a straight distance (H) from the center of the medical thread to the outer surface of the fixed portion 200 minus a thickness (d) of the cover cylinder 170 or the elastic thread cover tube 180. Then, the sloping jaw of the fixing groove can pull or fix the tissue during the insertion of the medical thread.

In this manner, even when the curves or grooves are pulled or stretched by the cover cylinder 170 or the elastic thread cover tube 180 placed around the outer circumference of the variable portion 300, the curves or grooves of the thread do not get in direction contact with the subcutaneous tissue but are stretched inside the cover cylinder, thereby preventing stimulation and damage of the tissue, and preventing pain in a treated area.

As described above, when utilizing the present invention, even if a medical suture without elasticity at a relatively low cost is used, by integrating the variable portion between the two fixed portions, the thread can naturally relax or contract with the movement of muscles (or tissues) after being buried under the subcutaneous tissue.

The lifting suture according to the present invention can naturally relax or contract with the movement of muscles since the variable portion 300 having the V-shaped or U-shaped elastic grooves is positioned between the spaced-apart fixed portions 200 formed on the outer surface of the medical thread, not due to the elasticity of the thread, and the basic alternating units including the variable portion and the fixed portions are repeated a certain number of times. Furthermore, the suture with the fixed portions 200 is made in an integrated structure, not in a separate combination structure, thereby having the unique effect of effectively contracting and relaxing in a stably fixed state.

In addition, the lifting suture according to the present invention can consistently maintain binding force with the skin due to the fixed portions 200, and have elastic recovery force due to the structural elasticity of the continuously formed variable portions 300 integrated with the fixed portions.

### [Sequence List Text]

100: variable lifting thread for pain reduction
110: first region
120: second region
130: fixing groove
140: sloping surface of fixing groove
150: acute angled sloping jaw of fixing groove
160: elastic groove 170: cover cylinder 180: elastic thread cover tube
200: fixed portion
300: variable portion

## Claims

1. A variable lifting thread for pain reduction, which is a medical thread for a lifting procedure,
wherein a medical thread (100) is formed to be partitioned in a length direction into a first region and a second region,
wherein the first region includes a fixed portion with fixing grooves having an acute angled sloping jaw formed on the outer surface of the medical thread to penetrate into the subcutaneous tissue to be fixed,
wherein the second region includes a variable portion with elastic grooves formed alternately (zig-zag) on both sides of the outer circumference of the medical thread to have structural elasticity, and
wherein when an external force acts, a connection portion between the elastic grooves of the variable portion stretches to provide elasticity to the medical thread.

2. The variable lifting thread according to claim 1, wherein between the variable portions of the second region, fixing grooves with the acute angled sloping jaws are formed to penetrate into the subcutaneous tissue to be fixed.

3. The variable lifting thread according to claim 1 or 2, wherein the fixing groove includes an inclined surface and the acute angled sloping jaw in an asymmetric structure.

4. The variable lifting thread according to claim 3, wherein concavely curved grooves are formed behind acute angled sloping jaw of the fixing groove to be spaced apart at a predetermined interval, and
wherein when the acute angled sloping jaw penetrates into the subcutaneous tissue, the curved grooves are curved and rotated, so that the acute angled sloping jaw further protrudes in an outer circumferential direction of the medical thread to penetrate deeper into the subcutaneous tissue and become more firmly fixed.

5. The variable lifting thread according to claim 1, wherein the variable portion is formed in a spring shape by being formed spirally in the length direction of the medical thread.

6. The variable lifting thread according to claim 1, wherein a depth (h) of the elastic groove is greater than a straight distance (H) from the center of the medical thread to the outer circumferential surface.

7. The variable lifting thread according to claim 1, wherein a cover cylinder is formed on the outer circumference of the variable portion.

8. The variable lifting thread according to claim 7, wherein the cover cylinder is formed into a tension spring-type elastic thread cover tube by being wound with an elastic thread.

9. The variable lifting thread according to claim 7 or 8, wherein the radius of the cover cylinder and the elastic thread cover tube is equal to or smaller than a radius (H) of the lifting thread.
